# EUROPÄISCHE PATENTSCHRIFT

(11) **EP 3 288 462 B1**
(45) Veröffentlichungstag und Bekanntmachung des Hinweises auf die Patenterteilung: **04.03.2020**
(21) Anmeldenummer: 16723745.2
(22) Anmeldetag: 19.05.2016
(51) Int. Cl.: A61B 6/03, A61B 6/06, A61B 6/00

(54) **RÖNTGENEINRICHTUNG FÜR DIE INVERSE COMPUTERTOMOGRAPHIE**
X-RAY DEVICE FOR INVERSE COMPUTER TOMOGRAPHY
APPAREIL DE RADIOGRAPHIE POUR LA TOMOGRAPHIE ASSISTÉE PAR ORDINATEUR DE TYPE INVERSÉ

(30) Priorität: 15.07.2015 DE 102015213285
(43) Veröffentlichungstag der Anmeldung: 07.03.2018
(73) Patentinhaber: Siemens Healthcare GmbH, 91052 Erlangen (DE)
(72) Erfinder: FREUDENBERGER, Jörg, 90562 Kalchreuth (DE); RÖHRER, Peter, 91056 Erlangen (DE)
(86) Internationale Anmeldenummer: PCT/EP2016/061260
(87) Internationale Veröffentlichungsnummer: WO 2017/008938

(56) Entgegenhaltungen:
- WO-A1-2014/047518
- WO-A1-2014/138500
- DE-A1-102009 007 857
- US-A1- 2014 226 787
- JONGDUK BAEK ET AL: "A multi-source inverse-geometry CT system: initial results with an 8 spot x-ray source array", PHYSICS IN MEDICINE AND BIOLOGY, INSTITUTE OF PHYSICS PUBLISHING, BRISTOL GB, Bd. 59, Nr. 5, 20. Februar 2014 (2014-02-20), Seiten 1189-1202, XP020257847, ISSN: 0031-9155, DOI: 10.1088/0031-9155/59/5/1189 [gefunden am 2014-02-20]
- HSIEH SCOTT S ET AL: "The feasibility of an inverse geometry CT system with stationary source arrays", MEDICAL PHYSICS, AIP, MELVILLE, NY, US, Bd. 40, Nr. 3, 1. März 2013 (2013-03-01), Seiten 31904-31904, XP012171044, ISSN: 0094-2405, DOI: 10.1118/1.4789918 [gefunden am 2013-02-14]

## Beschreibung

Die Erfindung betrifft eine Röntgeneinrichtung für die inverse Computertomographie, mit einer Mehrzahl von Röntgenstrahlern und einem den Röntgenstrahlern gegenüberliegend angeordneten Detektor. Die von den Röntgenstrahlern emittierten Röntgenstrahlen sind nach zumindest teilweiser Durchstrahlung eines im Zwischenbereich zwischen den Röntgenstrahlern und dem Detektor gelegenen Untersuchungsgebietes vom Detektor detektierbar.

Bei tomographischen Röntgenaufnahmen, wie insbesondere der Computertomographie oder der Tomosynthese, muss ein Untersuchungsobjekt aus unterschiedlichen Richtungen durchstrahlt werden, damit ein dreidimensionaler Bilddatensatz erzeugt werden kann. Dies erfolgt in der Regel mittels eines Röntgenstrahlers, der näherungsweise als punktförmige Röntgenquelle betrachtet werden kann, und eines relativ großen Detektors. Der Röntgenstrahler und der Detektor werden zur Erfassung von Bilddaten um das Untersuchungsobjekt rotiert.

Ferner wurden Röntgeneinrichtungen entwickelt, bei denen der Detektor aus Kostengründen verkleinert wurde. Bei derartigen Konfigurationen kann jedoch im Allgemeinen das gesamte zu erfassende Untersuchungsgebiet nicht mehr mit lediglich einem einzigen Röntgenstrahler vollständig ausgeleuchtet bzw. durchstrahlt werden.

EP 2 378 974 B1 schlägt daher eine Röntgeneinrichtung für die so genannte inverse Computertomographie vor, bei der lediglich ein Detektor mit einer relativ kleinen Detektorfläche vorgesehen ist. Zum Durchstrahlen des Untersuchungsgebiets sind mehrere Röntgenstrahler in einer äquidistanten Anordnung zueinander vorgesehen. Die Röntgenstrahler können sequentiell einzeln oder in Gruppen geschalten werden.

Aus Jongduk Baek et al, "A multi-source inverse-geometry CT system: initial results with a 8 spot x-ray source array", Physics in Medicine and Biology, Institute of _Physics Publishing, Bristol GB, Bd. 59, Nr. 5, Seiten 1189-1202, ist eine Röntgeneinrichtung für die inverse Computertomographie mit mehreren Röntgenstrahlern bekannt, die in zwei Reihen angeordneten sind. Die Röntgenstrahler einer jeweiligen Reihe sind zueinander äquidistant nebeneinander in Umfangsrichtung angeordnet. Die beiden Reihen sind voneinander in einer senkrecht zur Umfangsrichtung verlaufenden Richtung beabstandet und bilden somit eine zweidimensionale Anordnung von Röntgenstrahlern.

Aus WO 2014/138500 A1 ist eine weitere Röntgeneinrichtung für die Mammographie bekannt, bei der die Anordnung der Röntgenstrahler von einer linearen Anordnung in einer Reihe abweicht.

DE 10 2009 007857 A1 beschreibt eine Anode für einen Röntgenstrahler.

WO 2014/047518 A1 beschreibt stationäre Anordnungen von Röntgenstrahlern für die Computertomographie, die mehrere, in Umfangsrichtung beabstandete Untergruppen aufweisen. Die Röntgenstrahler jeder Untergruppe bestrahlen jeweils ein Gebiet, welches der gesamten Querschnittsfläche des Untersuchungsgebietes entspricht. Eine Anordnung mit ähnlicher Geometrie ist auch aus Hsieh Scott S. et al, "The feasability of an inverse geometry CT system with stationary source arrays", Medical Physics, AIP, Melville, NY, US, Bd. 40, Nr. 3, Seiten 31904-1 bis 31904-13 bekannt.

Ausgehend von diesem Stand der Technik ist es Aufgabe der vorliegenden Erfindung, eine verbesserte Röntgeneinrichtung anzugeben, die insbesondere hinsichtlich der Leistungsanforderung an die Röntgenstrahler optimiert ist.

Die Aufgabe wird erfindungsgemäß gelöst durch eine Röntgeneinrichtung der eingangs genannten Art mit den kennzeichnenden Merkmalen des Patentanspruches 1.

Vorteilhafte Ausgestaltungen der Erfindung sind Gegenstand der Unteransprüche.

Eine Röntgeneinrichtung für die inverse Computertomographie umfasst eine Mehrzahl von Röntgenstrahlern und einen den Röntgenstrahlern gegenüberliegend angeordneten Detektor. Die von den Röntgenstrahlern emittierten Röntgenstrahlen sind nach zumindest teilweiser Durchstrahlung eines im Zwischenbereich zwischen den Röntgenstrahlern und dem Detektor gelegenen Untersuchungsgebietes vom Detektor detektierbar. Erfindungsgemäß sind die Röntgenstrahler abweichend von einer äquidistanten Anordnung in einer Reihe nebeneinander in zumindest zwei voneinander beabstandete Unteranordnungen angeordnet, wobei jede Unteranordnung mehrere Röntgenstrahler umfasst. Ein Abstand der zumindest zwei Unteranordnungen voneinander ist größer als ein Abstand von benachbarten Röntgenstrahlern zumindest einer der Unteranordnungen.

Der Erfindung liegt folgende Beobachtung zu Grunde: Bei Konfigurationen, die eine gegenüber herkömmlichen Anordnungen verkleinerte Detektorfläche aufweisen, müssen mehrere Röntgenstrahler zum Durchleuchten des Untersuchungsgebietes vorgesehen werden, da nur der vom jeweiligen Röntgenstrahler bestrahlbare Raumwinkelbereich für die Bilderfassung genutzt werden kann, der auch vom Detektor erfasst wird. Im Ergebnis müssen daher im Allgemeinen umso mehr Röntgenstrahler vorgesehen werden, je kleiner die Detektorfläche des Detektors gewählt wird.

Der Detektor muss jedoch nach jeder Bestrahlung ausgelesen werden. Das bedeutet, dass jeder Röntgenstrahler einer linearen und in etwa äquidistanten Anordnung für eine Belichtung in etwa die gleiche Anzahl von Photonen abgeben müsste, wie der einzige Röntgenstrahler einer konventionellen Röntgeneinrichtung, um näherungsweise das gleiche Signal-RauschVerhältnis zu erreichen. Allerdings ist nun zur Aufnahme einer Projektion eine der Anzahl der Röntgenstrahlern entsprechende Anzahl von Belichtungen notwendig. Wenn man davon ausgeht, dass die Aufnahme einer Projektion zumindest näherungsweise innerhalb der gleichen Zeit erfolgen soll, so folgt hieraus, dass bei einer in etwa äquidistanten Anordnung von Röntgenstrahlern die von jedem der Röntgenstrahlern abzugebende Intensität in etwa um einen der Anzahl der Röntgenstrahlern entsprechenden Faktor erhöht sein muss. Dies hat unmittelbar Auswirkungen auf den Hochspannungsgenerator für die Röntgenstrahler, dessen Leistung um einen entsprechenden Faktor erhöht sein muss, und auf den Detektor, der um den entsprechenden Faktor schneller auslesbar sein muss.

An dieser Stelle wird noch darauf hingewiesen, dass sich die Belichtungszeiten zumindest im Bereich der medizinischen Anwendung auf Grund von störenden und Bildartefakte hervorrufenden Organbewegungen nicht beliebig verlängern lassen.

Es wurde erkannt, dass die vorstehend beschriebenen erhöhten Leistungsanforderungen an den Hochspannungsgenerator bei einer eindimensionalen, nicht äquidistanter Anordnung von Röntgenstrahlern lediglich im geringeren Ausmaß auftreten.

Gemäß der Erfindung wird daher vorgeschlagen, die Röntgenstrahler in zumindest zwei Unteranordnungen zu gruppieren. Jede der Unteranordnungen bestrahlt dabei im Wesentlichen ein Volumen des Untersuchungsgebiets von gleich großem Volumen. Dazu sind die Röntgenstrahler in den jeweiligen Unteranordnungen räumlich dicht angeordnet. Die Unteranordnungen sind in einem Abstand zueinander angeordnet, der größer ist, als ein Abstand von benachbarten Röntgenstrahlern in zumindest einer der Unteranordnungen.

Es hat sich gezeigt, dass bei einer derartigen Anordnung die Leistungsanforderungen an die einzelnen Röntgenstrahler und damit auch an den Hochspannungsgenerator für die Röntgenstrahler deutlich reduziert sind. Der Hochspannungsgenerator kann kleiner als bei anderen inversen Aufbauten dimensioniert werden, die eine unveränderte Belichtungszeit im Vergleich zu konventioneller Projektionsbildgebung anstreben. Die Dimensionierung hängt hierbei sowohl von der Anzahl der Untergruppen, in die die Röntgenstrahler gruppiert werden als auch von der angestrebten Belichtungszeit ab. Unter Annahme einer Belichtungszeit wie für den Fall einer konventionellen Anordnung, bei der nur ein Röntgenstrahler einen Detektor belichtet und Vernachlässigung von elektronischem Rauschen bei der Auslesung des Detektors muss beispielsweise bei einer Gruppierung in zwei Untergruppen der Hochspannungsgenerator nur etwa die doppelte Leistung im Vergleich zur konventionellen Anordnung abgeben. Entsprechend kann der Hochspannungsgenerator kleiner dimensioniert werden. Darüber hinaus ist der Einsatz von Röntgenstrahlern mit Stehanoden ermöglicht, da die von den Röntgenstrahlern abzugebende Intensität unterhalb eines kritischen Schwellwerts verbleibt, bei dem eine thermische Beschädigung der Anode zu erwarten wäre.

Die Gesamtheit der Röntgenstrahler ist in einer Reihe nebeneinander angeordnet. Da der Abstand der Untergruppen zueinander größer ist als der Abstand der Röntgenstrahler in zumindest einer der Untergruppen, bedeutet dies, dass die Gesamtheit der Röntgenstrahler nicht äquidistant angeordnet sind. Gemäß bevorzugten Ausführungsbeispielen sind die Röntgenstrahler in einer linearen Reihe oder auf einer Kreisbahn angeordnet.

Gemäß der Erfindung ist das von den Röntgenstrahlern der jeweiligen Unteranordnung bestrahlbare Gebiet auf ein Teilgebiet einer maximal durchstrahlbaren Querschnittsfläche des Untersuchungsgebietes begrenzt. Die Gesamtheit der Teilgebiete überdeckt die Querschnittsfläche des Untersuchungsgebietes vollständig.

Jedes der Teilgebiete ist in einem konkreten Ausführungsbeispiel randseitig von einer ersten und einer zweiten Geraden begrenzt, wobei die erste Gerade durch eine Seitenkante des Detektors, einen die Querschnittsfläche seitlich begrenzenden seitlichen Randpunkt und einen Röntgenstrahler der zugeordneten Unteranordnung verläuft. Die zweite Gerade verläuft durch eine weitere gegenüberliegend angeordnete Seitenkante des Detektors, einen die Querschnittsfläche oberseitig begrenzenden oberen Randpunkt und einen weiteren Röntgenstrahler der zugeordneten Unteranordnung.

Die zweiten Geraden der zumindest zwei Unteranordnungen dieses Ausführungsbeispiels schneiden sich vorzugsweise im oberen Randpunkt.

Vorzugsweise verlaufen die den zumindest zwei Unteranordnungen zugeordneten ersten Geraden näherungsweise parallel zueinander. In Weiterbildung der Erfindung ist daher vorgesehen, die Röntgenstrahler derart räumlich anzuordnen, dass die ersten Geraden der zumindest zwei Unteranordnungen in einem Winkel von weniger 30° zueinander verlaufen.

Jeder Unteranordnung ist besonders bevorzugt jeweils ein Kollimator zugeordnet, der den Strahlenverlauf entsprechend auf ein entsprechendes Teilgebiet des Untersuchungsgebietes begrenzt.

Die in den Unteranordnungen gruppierten Röntgenstrahler sind vorzugsweise innerhalb der zugehörigen Unteranordnung linear angeordnet.

Besonders bevorzugt sind die in den Unteranordnungen gruppierten Röntgenstrahler innerhalb der zugehörigen Unteranordnung äquidistant voneinander angeordnet.
In einem bevorzugten Ausführungsbeispiel sind genau zwei Unteranordnungen vorgesehen, die derart angeordnet sind, dass das Untersuchungsgebiet von der Gesamtheit der Röntgenstrahler der beiden Unteranordnungen vollständig bestrahlt werden kann. Bei einer geeigneten Anordnung der Röntgenstrahler und der Unteranordnungen kann die an die Röntgenstrahler gestellte erhöhte Leistungsanforderung bezüglich der zu emittierenden Intensität im Idealfall lediglich um einen Faktor von etwa zwei im Hinblick auf eine konventionelle Röntgeneinrichtung mit lediglich einer Röntgenquelle begrenzt werden. Bei solchen Ausführungen bestrahlen die beiden Unteranordnungen jeweils etwa die Hälfte des Volumens des Untersuchungsgebietes.

In einem Ausführungsbeispiel sind die zumindest zwei Unteranordnungen und der Detektor umfänglich um einen tunnelähnlichen Untersuchungsraum angeordnet. Eine derartige Ausbildung kann insbesondere zu einer Computertomographieeinrichtung mit inverser Geometrie korrespondieren. In einem anderen Ausführungsbeispiel sind die Röntgenstrahler im Wesentlichen plan angeordnet.

Wie bereits erwähnt, ermöglicht die vergleichsweise nur geringfügig zu erhöhende Strahlungsintensität in besonders vorteilhafter Weise die Verwendung von Röntgenstrahlern mit Stehanoden. Vorzugsweise weist jeder Röntgenstrahler der zumindest zwei Unteranordnungen eine Stehanode auf.

Besonders leistungsfähige Stehanoden bestehen zumindest teilweise aus Diamant und weisen eine Beschichtung aus Wolfram auf. Ein Einsatz derartiger Anoden hat sich als vorteilhaft erwiesen, da im Ergebnis die von den einzelnen Röntgenstrahlern zu emittierende Strahlungsintensität in jedem Fall gegenüber den herkömmlichen Röntgeneinrichtungen zu erhöhen ist.

Vorzugsweise ist die Stehanode zur besseren Abführung von Wärme in Kupfer eingebettet. Dies dient zur Vermeidung einer thermischen Beschädigung der Anode, so dass diese Röntgenstrahlung mit erhöhter Intensität bereitstellen kann.

Für eine weitere Beschreibung der Erfindung wird auf das in der Zeichnungsfigur gezeigte Ausführungsbeispiel verwiesen. Es zeigt in einer schematischen Prinzipskizze:
- Fig. 1: eine Schnittdarstellung der Röntgeneinrichtung gemäß einem Ausführungsbeispiel der Erfindung mit einer linearen Anordnung von Röntgenstrahlern;
- Fig. 2: eine Schnittdarstellung einer Röntgenstrahlung gemäß einem anderen Ausführungsbeispiel mit einer kreisbogenförmigen Anordnung von Röntgenstrahlern.

Einander entsprechende Teile sind in allen Figuren mit den gleichen Bezugszeichen versehen.

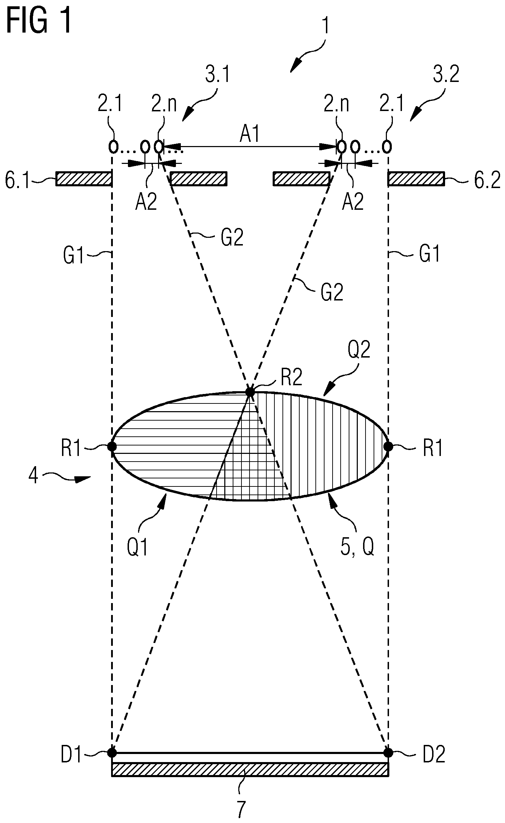

Figur 1 zeigt exemplarisch eine mögliche Ausführung der Röntgeneinrichtung 1 gemäß der Erfindung in einer schematischen Schnittdarstellung. Die Röntgeneinrichtung 1 umfasst mehrere Röntgenstrahler 2.1 bis 2.n, die nebeneinander angeordnet sind und in zwei Unteranordnungen 3.1, 3.2 gruppiert sind.

Die Gesamtheit der Röntgenstrahler 2.1 bis 2.n der Unteranordnungen in einer Reihe und nicht äquidistant zueinander angeordnet, da die Unteranordnungen 3.1, 3.2 einen größeren Abstand A1 voneinander aufweisen als der Abstand A2 der Röntgenstrahler in den jeweiligen Untergruppen 3.1, 3.2.

Die Röntgenstrahler 2.1 bis 2.n der zugehörigen Unteranordnungen 3.1, 3.2 sind in der dargestellten Zeichenebene in linearen Reihen und äquidistant zueinander angeordnet. Die Röntgenstrahler 2.1 bis 2.n haben voneinander innerhalb der entsprechenden Unteranordnung 3.1, 3.2 einen kleineren Abstand A2 voneinander, als die Unteranordnungen 3.1, 3.2 zueinander.
Die Röntgenstrahler 2.1 bis 2.n sind zumindest entsprechend der Untergruppen 3.1, 3.2 getrennt voneinander ansteuerbar. Vorzugsweise ist jeder der Röntgenstrahler 2.1 bis 2.n einzeln ansteuerbar.

In der senkrecht zur Zeichenebene verlaufenden Richtung können weitere Röntgenstrahler insbesondere randseitig an einem Untersuchungsraum 4 in einer abweichenden räumlichen Konfiguration vorgesehen sein.

Im zwischen den Röntgenstrahlern 2.1 bis 2.n und einem Detektor 7 gelegenen Untersuchungsraum 4 befindet sich ein Untersuchungsgebiet 5 (Engl.: region of interest), dessen Querschnittsfläche Q in der Zeichenebene näherungsweise ellipsenförmige Gestalt hat. Die Röntgenstrahler 2.1 bis 2.n der Unteranordnungen 3.1, 3.2 und der Detektor 7 sind so ausgerichtet, dass die von der Gesamtheit der Röntgenstrahlern 2.1 bis 2.n emittierbare und vom Detektor 7 erfassbare Röntgenstrahlung die Querschnittsfläche Q vollständig überdeckt.

Das von jeweils einer der Unteranordnung 3.1, 3.2 bestrahlbare Gebiet ist jedoch durch zugeordnete Kollimatoren 6.1, 6.2 auf Teilgebiete Q1, Q2 von etwa gleichem Volumen beschränkt. Jeder Kollimator 6.1, 6.2 ist dabei einer Unteranordnung 3.1, 3.2 und damit einer Vielzahl von Röntgenstrahlern 2.1 bis 2.n zugeordnet.

Das Teilgebiet Q1 ist von der Unteranordnung 3.1 bestrahlbar und ist in der gezeigten Zeichenebene von ersten und zweiten Geraden G1, G2 und dem Rand der Querschnittsfläche Q des Untersuchungsgebietes 5 begrenzt. Entsprechend ist das Teilgebiet Q2 randseitig von den ersten und zweiten Geraden G1, G2, die der Unteranordnung 3.2 zugeordnet sind, und dem Rand der Querschnittsfläche Q begrenzt.

Die der Unteranordnung 3.1 zugeordnete erste Gerade G1 berühren dabei jeweils den Rand der Querschnittsfläche Q in einem seitlichen Randpunkten R1. Die ersten Geraden G1 schneiden ferner erste Seitenkanten D1 des Detektors 7 und verlaufen durch einen ersten Röntgenstrahler 2.1 der Unteranordnung 3.1. Die zweite Gerade G2 verläuft von einem weiteren Röntgenstrahler 2.n der Unteranordnung 3.1 durch einen die Querschnittsfläche Q oberseitig begrenzenden oberen Randpunkt R2 zu einer zweiten Seitenkante D2 des Detektors 7, die der ersten Seitenkante D1 gegenüberliegt.

Die Unteranordnung 3.2 ist bezüglich einer Mittellängsachse der Röntgeneinrichtung 1 spiegelsymmetrisch aufgebaut, so dass sich die zweiten Geraden G2 der Unteranordnungen 3.1, 3.2 im oberen Randpunkt R2 schneiden.

Die ersten Geraden G1, der beiden Unteranordnungen 3.1, 3.2 verlaufen nahezu parallel zueinander. Im gezeigten Beispiel ist die Abweichung deutlich weniger als +/- 15°.
Die Röntgenstrahler 2.1 bis 2.n weisen in nicht näherdargestellter Weise Stehanoden auf, die zumindest teilweise aus mit Wolfram beschichtetem Diamant bestehen. Die Stehanode ist ferner in Kupfer eingebettet.

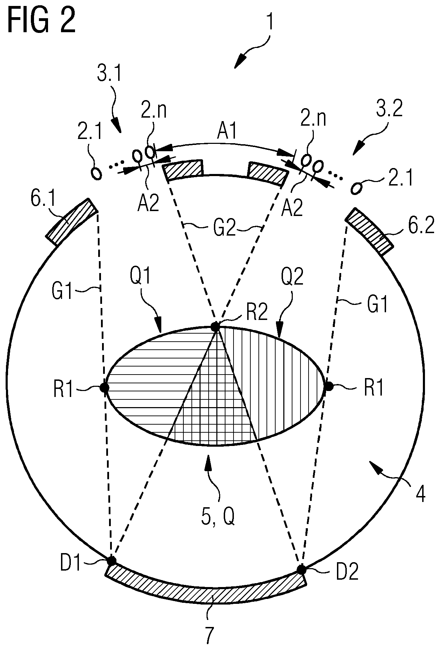

Figur 2 zeigt ein weiteres Ausführungsbeispiel der Erfindung, bei dem die Röntgenstrahler 2.1 bis 2.n umfänglich um einen tunnelähnlichen Untersuchungsraum 5 angeordnet sind. Das Ausführungsbeispiel des zweiten Ausführungsbeispiels unterscheidet sich von dem in Figur 1 gezeigten ersten Ausführungsbeispiel nur insoweit, dass die Röntgenstrahler 2.1 bis 2.n entlang einer Kreisbogenlinie angeordnet sind. Hinsichtlich der übrigen Merkmale, insbesondere hinsichtlich der Abstände A1, A2 der in Untergruppen 3.1, 3.2 angeordneten Röntgenstrahlern 2.1 bis 2.n wird auf die Ausführungen mit Bezug auf Figur 1 verwiesen.
In einem weiteren Ausführungsbeispiel sind die Röntgenstrahler 2.1 bis 2.n, wie in Figur 2 gezeigt, nebeneinander und umfänglich um den Untersuchungsraum 5 angeordnet. Zusätzlich sind weitere Röntgenstrahler 2.1 bis 2.n in axialer Richtung, also senkrecht zur Zeichenebene der Figur 2 angeordnet, deren Anordnung der in Figur 1 angegebenen Konfiguration entspricht.
Obwohl die Erfindung im Detail durch das bevorzugte Ausführungsbeispiel näher illustriert und beschrieben wurde, so ist die Erfindung nicht durch die in den Figuren gezeigten Ausführungsbeispiele eingeschränkt.

## Patentansprüche

1. Röntgeneinrichtung (1) für eine inverse Computertomographiekonfiguration, welche eine Mehrzahl von Röntgenstrahlern (2.1 bis 2.n) und einen den Röntgenstrahlern (2.1 bis 2.n) gegenüberliegend angeordneten Detektor (7) umfasst, wobei die von den Röntgenstrahlern (2.1 bis 2.n) emittierten Röntgenstrahlen nach zumindest teilweiser Durchstrahlung eines im Zwischenbereich zwischen den Röntgenstrahlern (2.1 bis 2.n) und dem Detektor (7) gelegenen Untersuchungsgebietes (5) vom Detektor (7) detektierbar sind, wobei die Röntgenstrahler (2.1 bis 2.n) abweichend von einer äquidistanten Anordnung angeordnet sind, wobei die Röntgenstrahler (2.1 bis 2.n) in einer Reihe nebeneinander in zumindest zwei voneinander beabstandeten Unteranordnungen (3.1, 3.2) angeordnet sind, wobei jede Unteranordnung (3.1, 3.2) mehrere Röntgenstrahler (2.1 bis 2.n) umfasst und ein Abstand der zumindest zwei Unteranordnungen (3.1) voneinander größer ist als ein Abstand von benachbarten Röntgenstrahlern (2.1 bis 2.n) zumindest einer der Unteranordnungen (3.1, 3.2), **dadurch gekennzeichnet, dass** jeder Unteranordnung (3.1, 3.2) jeweils ein Kollimator (6.1, 6.2) zugeordnet ist, der das von den Röntgenstrahlern (2.1 bis 2.n) der jeweiligen Unteranordnung (3.1, 3.2) bestrahlbare Gebiet auf ein Teilgebiet (Q1, Q2) einer maximal durchstrahlbaren Querschnittsfläche (Q) des Untersuchungsgebietes (5) begrenzt, wobei die Gesamtheit der Teilgebiete (Q1, Q2) die Querschnittsfläche (Q) des Untersuchungsgebietes (5) vollständig überdeckt.

2. Röntgeneinrichtung (1) nach Anspruch 1, **dadurch gekennzeichnet, dass** jedes der Teilgebiete (Q1, Q2) randseitig von einer ersten und einer zweiten Geraden (G1, G2) begrenzt ist, wobei die erste Gerade (G1) durch eine Seitenkante (D1, D2) des Detektors (7), einen die Querschnittsfläche (Q) seitlich begrenzenden seitlichen Randpunkt (R1) und einen Röntgenstrahler (2.1) der zugeordneten Unteranordnung (3.1, 3.2) verläuft und die zweite Gerade (G2) durch eine weitere gegenüberliegende Seitenkante (D1, D2) des Detektors (7), einer die Querschnittsfläche (Q) oberseitig begrenzenden oberen Randpunkt (R2) und einem weiteren Röntgenstrahler (2.n) der der zugeordneten Unteranordnung (3.1, 3.2) verläuft.

3. Röntgeneinrichtung (1) nach Anspruch 2, **dadurch gekennzeichnet, dass** sich die zweiten Geraden (G2) der zumindest zwei Unteranordnungen (3.1, 3.2) im oberen Randpunkt (R2) schneiden.

4. Röntgeneinrichtung (1) nach Anspruch 2 oder 3, **dadurch gekennzeichnet, dass** die den zumindest zwei Unteranordnungen (3.1, 3.2) zugeordneten ersten Geraden (G1) zueinander in einem Winkel von weniger als 30° verlaufen.

5. Röntgeneinrichtung (1) nach einem der vorhergehenden Ansprüche, **dadurch gekennzeichnet, dass** die in den Unteranordnungen (3.1, 3.2) gruppierten Röntgenstrahler (2.1 bis 2.n) linear angeordnet sind.

6. Röntgeneinrichtung (1) nach einem der vorhergehenden Ansprüche, **dadurch gekennzeichnet, dass** die in den Unteranordnungen (3.1, 3.2) gruppierten Röntgenstrahler (2.1 bis 2.n) äquidistant voneinander angeordnet sind.

7. Röntgeneinrichtung (1) nach einem der vorhergehenden Ansprüche, **dadurch gekennzeichnet, dass** genau zwei Unteranordnungen (3.1, 3.2) vorgesehen sind.

8. Röntgeneinrichtung (1) nach einem der vorhergehenden Ansprüche, **dadurch gekennzeichnet, dass** die Röntgenstrahler (2.1 bis 2.n) der zumindest zwei Unteranordnungen (3.1, 3.2) und der Detektor (7) umfänglich um einen tunnelähnlichen Untersuchungsraum (4) angeordnet sind.

9. Röntgeneinrichtung (1) nach einem der vorhergehenden Ansprüche, **dadurch gekennzeichnet, dass** jeder Röntgenstrahler (2.1 bis 2.n) der zumindest zwei Unteranordnungen (3.1, 3.2) eine Stehanode aufweist.

10. Röntgeneinrichtung (1) nach Anspruch 9, **dadurch gekennzeichnet, dass** die Stehanode zumindest teilweise aus Diamant besteht und eine Beschichtung aus Wolfram aufweist.

11. Röntgeneinrichtung (1) nach Anspruch 9 oder 10, **dadurch gekennzeichnet, dass** die Stehanode in Kupfer eingebettet ist.

## Claims

1. X-ray device (1) for an inverse computed tomography configuration, which includes a plurality of X-ray emitters (2.1 to 2.n) and a detector (7) arranged opposite the X-ray emitters (2.1 to 2.n), wherein after passing through at least some of a region of interest (5) located in the intermediate region between the X-ray emitters (2.1 to 2.n) and the detector (7), the X-rays emitted by the X-ray emitters (2.1 to 2.n) can be detected by the detector (7), wherein the X-ray emitters (2.1 to 2.n) are arranged in an arrangement other than equidistant, wherein the X-ray emitters (2.1 to 2.n) are arranged in series with one another, in at least two mutually spaced sub-arrangements (3.1, 3.2), wherein each sub-arrangement (3.1, 3.2) includes a plurality of X-ray emitters (2.1 to 2.n), and a spacing between the at least two sub-arrangements (3.1) is greater than a spacing between adjacent X-ray emitters (2.1 to 2.n) of at least one of the sub-arrangements (3.1, 3.2), **characterised in that** associated with each sub-arrangement (3.1, 3.2) is a respective collimator (6.1, 6.2), which limits the region that can be irradiated by the X-ray emitters (2.1 to 2.n) of the respective sub-arrangement (3.1, 3.2) to a partial region (Q1, Q2) of a maximum cross-sectional area (Q) of the region of interest (5) that can be irradiated, wherein the partial regions (Q1, Q2), taken in their entirety, completely cover the cross-sectional area (Q) of the region of interest (5).

2. X-ray device (1) according to claim 1, **characterised in that** each of the partial regions (Q1, Q2) is delimited at its margin by a first and a second straight line (G1, G2), wherein the first straight line (G1) runs through a side edge (D1, D2) of the detector (7), a lateral marginal point (R1) that laterally delimits the cross-sectional area (Q), and an X-ray emitter (2.1) of the associated sub-arrangement (3.1, 3.2), and the second straight line (G2) runs through a further, opposite side edge (D1, D2) of the detector (7), an upper marginal point (R2) that upwardly delimits the cross-sectional area (Q), and a further X-ray emitter (2.n) of the associated sub-arrangement (3.1, 3.2).

3. X-ray device (1) according to claim 2, **characterised in that** the second straight lines (G2) of the at least two sub-arrangements (3.1, 3.2) intersect at the upper marginal point (R2).

4. X-ray device (1) according to claim 2 or 3, **characterised in that** the first straight lines (G1) that are associated with the at least two sub-arrangements (3.1, 3.2) run at an angle of less than 30° to one another.

5. X-ray device (1) according to one of the preceding claims, **characterised in that** the X-ray emitters (2.1 to 2.n) that are grouped in the sub-arrangements (3.1, 3.2) are arranged linearly.

6. X-ray device (1) according to one of the preceding claims, **characterised in that** the X-ray emitters (2.1 to 2.n) that are grouped in the sub-arrangements (3.1, 3.2) are arranged to be equidistant from one another.

7. X-ray device (1) according to one of the preceding claims, **characterised in that** exactly two sub-arrangements (3.1, 3.2) are provided.

8. X-ray device (1) according to one of the preceding claims, **characterised in that** the X-ray emitters (2.1 to 2.n) of the at least two sub-arrangements (3.1, 3.2) and the detector (7) are arranged peripherally around a tunnel-like examination chamber (4).

9. X-ray device (1) according to one of the preceding claims, **characterised in that** each X-ray emitter (2.1 to 2.n) of the at least two sub-arrangements (3.1, 3.2) has a stationary anode.

10. X-ray device (1) according to claim 9, **characterised in that** the stationary anode is made at least partly of diamond and has a coating of tungsten.

11. X-ray device (1) according to claim 9 or 10, **characterised in that** the stationary anode is embedded in copper.

## Revendications

1. Dispositif (1) de radiographie pour une configuration de tomographie assistée par ordinateur de type inversé, qui comprend une pluralité d'émetteurs (2.1 à 2.n) de rayons X et un détecteur (7) monté en face des émetteurs (2.1 à 2.n) de rayons X, les rayons X émis par les émetteurs (2.1 à 2.n) de rayons X pouvant, après avoir traversé, au moins en partie, un domaine (5) à examiner, mis dans la région intermédiaire entre les émetteurs (2.1 à 2.n) de rayons X et le détecteur (7), être détectés par le détecteur (7), les émetteurs (2.1 à 2.n) de rayons X étant disposés en s'écartant d'un agencement équidistant, les émetteurs (2.1 à 2.n) de rayons X étant disposés les uns à côté des autres suivant une rangée, en au moins deux sous-agencements (3.1, 3.2) à distance l'un de l'autre, chaque sous-agencement (3.1, 3.2) comprenant plusieurs émetteurs (2.1 à 2.n) de rayons X et une distance entre les au moins deux sous-agencements (3.1) l'un par rapport à l'autre étant plus grande qu'une distance entre des émetteurs (2.1 à 2.n) de rayons X voisins d'au moins l'un des sous-agencements (3.1, 3.2), **caractérisé en ce qu'**à chaque sous-agencement (3.1, 3.2) est associé, respectivement, un collimateur (6.1, 6.2), qui délimite le domaine pouvant être soumis à rayonnement par les émetteurs (2.1 à 2.n) de rayons X du sous-agencement (3.1, 3.2) respectif à un domaine (Q1, Q2) partiel d'une surface (Q) de section transversale, pouvant être traversée par du rayonnement au maximum, du domaine (5) à examiner, l'ensemble des domaines (Q1, Q2) partiels recouvrant entièrement la surface (Q) de section transversale du domaine (5) à examiner.

2. Dispositif (1) à rayons X suivant la revendication 1, **caractérisé en ce que** chacun des domaines (Q1, Q2) partiels est délimité du côté du bord par une première et par une deuxième droites (G1, G2), la première droite (G1) passant par un bord (D1, D2) latéral du détecteur (7), par un point (R1) de bord latéral délimitant latéralement la surface (Q) de section transversale et par un émetteur (2.1) de rayons X du sous-agencement (3.1, 3.2) associé, et la deuxième droite (G2) passant par un autre bord (D1, D2) latéral opposé du détecteur (7), par un point (R2) de bord supérieur délimitant du côté supérieur la surface (Q) de section transversale et par un autre émetteur (2.n) de rayons X du sous-agencement (3.1, 3.2) associé.

3. Dispositif (1) à rayons X suivant la revendication 2, **caractérisé en ce que** les deuxièmes droites (G2) des au moins deux sous-agencements (3.1, 3.2) se coupent au point (R2) de bord supérieur.

4. Dispositif (1) à rayons X suivant la revendication 2 ou 3, **caractérisé en ce que** les premières droites (G1), associées aux au moins deux sous-agencements (3.1, 3.2), font entre elles un angle de moins de 30°.

5. Dispositif (1) à rayons X suivant l'une des revendications précédentes, **caractérisé en ce que** les émetteurs (2.1 à 2.n) de rayons X, regroupés dans les sous-agencements (3.1, 3.2), sont disposés linéairement.

6. Dispositif (1) à rayons X suivant l'une des revendications précédentes, **caractérisé en ce que** les émetteurs (2.1 à 2.n) de rayons X, regroupés dans les sous-agencements (3.1, 3.2), sont équidistants les uns des autres.

7. Dispositif (1) à rayons X suivant l'une des revendications précédentes, **caractérisé en ce qu'**il est prévu exactement deux sous-agencements (3.1, 3.2).

8. Dispositif (1) à rayons X suivant l'une des revendications précédentes, **caractérisé en ce que** les émetteurs (2.1 à 2.n) de rayons X des au moins deux sous-agencements (3.1, 3.2) et le détecteur (7) sont disposés autour d'un espace (4) d'examen analogue à un tunnel.

9. Dispositif (1) à rayons X suivant l'une des revendications précédentes, **caractérisé en ce que** chaque émetteur (2.1 à 2.n) de rayons X des au moins deux sous-agencements (3.1, 3.2) a une anode verticale.

10. Dispositif (1) à rayons X suivant la revendication 9, **caractérisé en ce que** l'anode verticale est, au moins en partie, en diamant et a un revêtement en tungstène.

11. Dispositif (1) à rayons X suivant la revendication 9 ou 10, **caractérisé en ce que** l'anode verticale est incorporée dans du cuivre.
